# EUROPEAN PATENT SPECIFICATION

(11) **EP 0 352 826 B1**
(45) Date of publication and mention of the grant of the patent: **26.10.1994**
(21) Application number: 89201113.1
(22) Date of filing: 01.05.1989
(51) Int. Cl.: A61K 31/60

(54) **Use of 5-aminosalicylic acid in the treatment of dermatological disorders**
Verwendung von 5-Amino-salicylsäure für die Behandlung von Hautkrankheiten
Utilisation de l'acide 5-aminosalicylique pour le traitement des maladies de la peau

(30) Priority: 05.05.1988 EP 88200899
(43) Date of publication of application: 31.01.1990
(73) Proprietor: TILLOTTS PHARMA AG, 4417 Ziefen (CH)
(72) Inventor: Olthoff, Margaretha, NL-2286 HA Rijswijk (NL); Hulsmans, Robertus Franciscus Henricus Johannes, NL-6226 HA Maastricht (NL)
(74) Representative: Burford, Anthony Frederick

(56) References cited:
- EP-A- 0 291 159
- Br. J. Dermatol 120 (3), March 1989, 471-472

## Description

The invention relates to 5-aminosalicylic acid (5-ASA) used in a new pharmaceutical formulation.

Among the compounds used in dermatology, corticosteroids are widely used for treating a great variety of diseases characterized by inflammation of acute, subacute and chronic types and hyperproliferations of the epidermis. Topical cortico-steroids, while being very active anti-inflammatory compounds, all have grave disadvantages, particularly in that they are contra-indicated in inflammatory and/or proliferative dermatoses (such as acne, dermatitis, psoriasis and psoriasis pustulosa) which are secondarily infected by microorganisms, because they favour the overgrowth of microorganisms.

They cannot be used either in dermatoses characterized by loss of skin with or without inflammation such as bullous dermatoses, pyoderma gangrenosum and inflamed or non-inflamed ulcerative dermatoses, chronic ulcers and decubitus.

Moreover most of the topical corticosteroids have local adverse effects in longstanding therapy and may have systemic adverse effects in treatments which involve extensive skin surfaces and/or longer periods of application.

Furthermore topical corticosteroids have no analgesic properties.

On the other hand non-steroidal anti-inflammatory topical substances, among which are coal tar and wood tar, while being generally free of the above local and systemic side effects and sometimes possessing analgesic properties, are much less effective. Furthermore these compounds cannot be used in pyoderma-like conditions and they do not promote granulation and wound healing.

5-Aminosalicylic acid is a known compound, currently used in the therapy of chronic inflammatory bowel diseases such as Crohn's Disease and ulcerative colitis. For these indications 5-aminosalicylic acid is always administered by the oral or rectal route.

The prior, not prepublished, EP-A-0291 159 discloses pharmaceutical compositions comprising 5-ASA and related compounds for use in the treatment of proliferative skin diseases such as psoriasis and various types of dermatitis.

It has now been found, that 5-ASA can also be used with excellent results in the treatment of severe dermatological disorders associated with therapy-resistant loss of epithelium, such as pyoderma gangrenosum, leg ulcers, mouth ulcers, decubitus, bullous dermatoses and burns.

For these dermatological indications 5-ASA is to be applied topically. In principle any of the type of carriers known in the art, such as ointments, creams, gels, lotions, powders and suspensions, can be applied topically with 5-ASA according to the invention.

The invention therefore concerns the use of 5-aminosalicylic acid in the preparation of topical pharmaceutical formulations for the treatment of dermatological disorders associated with loss of epithelium.

5-ASA is known to be rather unstable. Difficulties in formulating it into stable pharmaceutical topical preparations were therefore to be expected. However, 5-ASA was found to be relatively easily compatible with several formulations of known types. It was, however, observed that 5-ASA is incompatible with polyethylene glycols.

The concentration of 5-ASA in the topical formulations according to the invention is suitably between 0.1 - 25% by weight, preferably between 1 - 20% by weight, and more preferably between 2 - 10% by weight.

The following examples illustrate the invention.

### Example 1

### 5-ASA lipocream (5%)

190 g of white soft paraffin, 90 g of liquid paraffin, 30 g of cetostearyl alcohol and 15 g of polyethyleneglycol-1000-monocetyl ether (CETOMACROGOL 1000), 25 g of 5-ASA and 0.75 g of ascorbyl palmitate were melted and heated until a temperature of 70-80°C was reached. 1 g of methyl hydroxybenzoate and 0.75 g of ascorbic acid were added to 147.5 g of water (de-ionized). While stirring this mixture was heated until all compounds were dissolved. After allowing to cool, the solution was adjusted to a pH of about 4 with a mixture of sodium phosphates. While stirring, this solution was heated in a suitable ointment mixer to 70-80°C. To this solution the prepared liquid fatty composition at a temperature of 70-80°C was added. After vigorous mixing, under reduced pressure, the mass was gently stirred until cool.

### Examples 2-4

### 5-ASA lipocream (2%, 10% and 20%)

Similarly to Example 1, lipocreams containing the following ingredients were prepared:

| Ingredients (grams) | 5-ASA-lipocreams | | |
|---|---|---|---|
| | 2% | 10% | 20% |
| 5-ASA | 10.00 | 50.00 | 100.00 |
| White soft paraffin | 196.03 | 179.94 | 159.84 |
| Liquid paraffin | 92.86 | 85.24 | 75.71 |
| Cetostearyl alcohol | 30.95 | 28.41 | 25.24 |
| CETOMACROGOL 1000 | 15.48 | 14.20 | 12.62 |
| Ascorbyl palmitate | 0.75 | 0.75 | 0.75 |
| Methyl hydroxybenzoate | 1.00 | 1.00 | 1.00 |
| Ascorbic acid | 0.75 | 0.75 | 0.75 |
| Water | 152.18 | 139.70 | 124.09 |

### Example 5

### 5-ASA gel (5%)

100 mg of disodium edetate was dissolved in 35 ml of water. To this solution 10 g of propylene glycol was added. In a mortar 1 g of CARBOPOL 94™ (carbomer) and 5 g of 5-ASA were ground and mixed together, and, when a homogeneous mixture was obtained, intimately mixed with the forementioned solution. Then slowly, while stirring, a solution of 1 g triethanolamine in 11 ml of water was added to the mass and thereafter, in small portions, a solution of ascorbic acid in 42 ml of water.

### Examples 6-8

### 5-ASA gel (2%, 10% and 20%)

Similarly to Example 5, gels containing the following ingredients were prepared:

| Ingredients (grams) | 5-ASA-lipocreams | | |
|---|---|---|---|
| | 2% | 10% | 20% |
| 5-ASA | 2.00 | 10.00 | 20.00 |
| Disodium edetate | 0.10 | 0.10 | 0.10 |
| CARBOPOL 94™ | 2.15 | 1.12 | 1.00 |
| Propylene glycol | 9.80 | 9.00 | 8.00 |
| Triethanolamine | 1.65 | 0.95 | 0.85 |
| Ascorbic acid | 0.50 | 0.50 | 0.50 |
| Water | 83.40 | 78.33 | 69.55 |

### Example 9

### 5-ASA (drying) wound lotion (5%)

23 g of an aqueous stock solution of VISCONTRAN MHPC 6000™ (methylhydroxypropylcellulose) (2.2%) was diluted with 61.6 of de-ionized water, containing 0.5 g of ascorbic acid. In a mortar 5 g of 5-ASA and 10 g LEVILITE™ (silica) were ground and mixed together and thereafter this mixture was dispersed in the lotion by means of an Ultra Turrax mixer.

### Example 10

### 5-ASA emulsion lotion (5%)

10 g of TEFFOSE 63™ (PEG-6-32 stearate and glycolstearate) and 5 g of LABRAFAC LIPO™ (glyceryl tricaprylocaprate) were melted at a temperature of 80°C. 50 ml of de-ionized water was heated to 80°C and then added the melted fatty mass. While stirring this warm cream-mass, 5 g of 5-ASA was added. The mass was gently stirred until cool. Stirring was discontinued for about 15 minutes. After this pause, while stirring, 30 g of water containing 150 mg of ascorbic acid was added to the mass and stirring was continued for 30 minutes.

### Example 11

### 5-ASA drying paste (20%)

2 g of 5-ASA and 2 g of LEVILITE™ were ground and mixed together in a mortar. 10 ml of water was added to the powder mixture and intimately mixed with it.

### Example 12

### 5-ASA emulsion lotion (5%)

10 g of TEFFOSE 63™, 2 g of LABRAFIL 1944™ (unsaturated polyglycolyzed glyceride) and 8 g of LABRAFAC LIPO™ were melted at a temperature of 80°C. 2 g of UCARE JR 125™ (cationic cellulose-polymer) was dispersed in 50 ml of water, for 15 minutes, and subsequently heated to 80°C. 0.15 g of methyl hydroxybenzoate and 0.075 g of propyl hydroxybenzoate were dissolved in this solution, and also 0.15 g of ascorbic acid. Then this solution was added to the melted fatty mass, while stirring. Before starting to cool, 5 g of 5-ASA was added (stirring continued). When the mass reached a temperature of about 25°C, stirring was discontinued for 15 minutes. After this pause, 23 g of water was added and stirring was carried on for 30 minutes.

### Example 13

### Clinical result of treatment of gangrenous pyoderma with 5-ASA

A patient suffering from a painful fresh pyodermal gangrenous leg ulcer, measuring 3 cm across, was treated with a 20% W/W dispersion of 5-ASA in saline. The ulcer was covered with a gauze, which was soaked with the above dispersion. This ulcer healed completely after a total of 16 treatments (the first 10 in a frequency of three times a week, followed by 4 in a frequency of twice a week and finally 2 in a frequency of once a week). The pain disappeared after the very first treatment.

## Claims

1. The use of 5-aminosalicylic acid in the preparation of a topical pharmaceutical formulations for the treatment of a dermatological disorder associated with loss of epithelium other than acne, eczema or psoriasis.

2. A use as claimed in Claim 1, wherein said concentration of 5-aminosalicylic acid in said formulation is 0.1 - 25% by weight.

3. A use as claimed in Claim 2, wherein said concentration is 2 - 10% by weight.

4. A use as claimed in any one of Claims 1 to 3, wherein said formulation is for the treatment of pyoderma gangrenosum.

5. A use as claimed in any one of Claims 1 to 3, wherein said formulation is for the treatment of leg ulcers.

6. A use as claimed in any one of Claims 1 to 3, wherein said formulation is for the treatment of inflamed ulcerative dermatosis.

7. A use as claimed in any one of Claims 1 to 3, wherein said formulation is for the treatment of non-inflamed ulcerative dermatosis.

8. A use as claimed in any one of Claims 1 to 3, wherein said formulation is for the treatment of chronic ulcers.

9. A use as claimed in any one of Claims 1 to 3, wherein said formulation is for the treatment of decubitus.

10. A use as claimed in any one of Claims 1 to 3, wherein said formulation is for the treatment of bullous dermatoses.

11. A use as claimed in any one of Claims 1 to 3, wherein said formulation is for the treatment of burns.

12. A use as claimed in any one of the preceding claims, wherein said formulation is an ointment, cream, gel or powder.

13. A use as claimed in any one of Claims 1 to 11, wherein said formulation is a lotion or suspension.

## Patentansprüche

1. Verwendung von 5-Aminosalicylsäure bei der Herstellung von topischen pharmazeutischen Formulierungen zur Behandlung einer dermatologischen Erkrankung, die mit Epithelverlust einhergeht, unter Ausschluß von Akne, Ekzemen oder Psoriais.

2. Verwendung nach Anspruch 1, bei der die Konzentration an 5-Aminosalicylsäure in der Formulierung 0,1 - 25 Gew.-% beträgt.

3. Verwendung nach Anspruch 2, bei der die Konzentration 2 - 10 Gew.-% beträgt.

4. Verwendung nach einem der Ansprüche 1 bis 3, bei der die Formulierung die Behandlung von Pyoderma gangraenosum betrifft.

5. Verwendung nach einem der Ansprüche 1 bis 3, bei der die Formulierung die Behandlung von Beingeschwüren betrifft.

6. Verwendung nach einem der Ansprüche 1 bis 3, bei der die Formulierung die Behandlung einer entzündeten ulzerösen Dermatose betrifft.

7. Verwendung nach einem der Ansprüche 1 bis 3, bei der die Formulierung die Behandlung einer nicht entzündeten ulzerösen Dermatose betrifft.

8. Verwendung nach einem der Ansprüche 1 bis 3, bei der die Formulierung die Behandlung chronischer Geschwüre betrifft.

9. Verwendung nach einem der Ansprüche 1 bis 3, bei der die Formulierung die Behandlung von Dekubitus betrifft.

10. Verwendung nach einem der Ansprüche 1 bis 3, bei der die Formulierung die Behandlung von bullösen Dermatosen betrifft.

11. Verwendung nach einem der Ansprüche 1 bis 3, bei der die Formulierung die Behandlung von Verbrennungen betrifft.

12. Verwendung nach einem der vorhergehenden Ansprüche, bei der die Formulierung eine Salbe, eine Creme, ein Gel oder ein Pulver ist.

13. Verwendung nach einem der Ansprüche 1 bis 11, bei der die Formulierung eine Lotion oder Suspension ist.

## Revendications

1. Utilisation de l'acide 5-aminosalicylique dans la préparation de formulations pharmaceutiques topiques pour le traitement d'un trouble dermatologique associé à une perte d'épithélium autre que l'acné, l'eczéma et le psoriasis.

2. Utilisation selon la revendication 1, dans laquelle la concentration de l'acide 5-aminosalicylique dans ladite formulation est 0,1-25% en poids.

3. Utilisation selon la revendication 2, dans laquelle ladite concentration est 2-10% en poids.

4. Utilisation selon l'une quelconque des revendications 1 à 3, dans laquelle ladite formulation est pour le traitement de la pyodermie gangréneuse.

5. Utilisation selon l'une quelconque des revendications 1 à 3, dans laquelle ladite formulation est pour le traitement des ulcères de jambe.

6. Utilisation selon l'une quelconque des revendications 1 à 3, dans laquelle ladite formulation est pour le traitement de la dermatose ulcérative enflammée.

7. Utilisation selon l'une quelconque des revendications 1 à 3, dans laquelle ladite formulation est pour le traitement de la dermatose ulcérative non enflammée.

8. Utilisation selon l'une quelconque des revendications 1 à 3, dans laquelle ladite formulation est pour le traitement des ulcères chroniques.

9. Utilisation selon l'une quelconque des revendications 1 à 3, dans laquelle ladite formulation est pour le traitement des escarres du décubitus.

10. Utilisation selon l'une quelconque des revendications 1 à 3, dans laquelle ladite formulation est pour le traitement des dermatoses bulleuses.

11. Utilisation selon l'une quelconque des revendications 1 à 3, dans laquelle ladite formulation est pour le traitement des brûlures.

12. Utilisation selon l'une quelconque des revendications précédentes dans laquelle ladite formulation est un onguent, une crème, un gel ou une poudre.

13. Utilisation selon l'une quelconque des revendications 1 à 11, dans laquelle ladite formulation est une lotion ou une suspension.
